# EUROPEAN PATENT SPECIFICATION

(11) **EP 3 060 261 B1**
(45) Date of publication and mention of the grant of the patent: **20.03.2019**
(21) Application number: 14806433.0
(22) Date of filing: 24.10.2014
(51) Int. Cl.: A61L 2/07, A61L 2/20, A61L 2/26

(54) **HYDRAULIC COMPONENT AND METHOD FOR IMPROVING THE HYGIENIC AND SANITARY CHARACTERISTICS OF A HYDRAULIC COMPONENT**
HYDRAULISCHE KOMPONENTE UND VERFAHREN ZUR VERBESSERUNG DER HYGIENISCHE UND SANITÄRE EIGENSCHAFTEN EINES HYDRAULISCHEN KOMPONENTE
COMPOSANT HYDRAULIQUE ET PROCEDE POUR AMELIORER LES CARACTERISTIQUES HYGIÉNIQUES ET SANITAIRES D'UN COMPOSANT HYDRAULIQUE

(30) Priority: 25.10.2013 IT BO20130590
(43) Date of publication of application: 31.08.2016
(73) Proprietor: Marini, Giuseppe, 40064 Ozzano dell'Emilia (IT); Muggiasca, Andrea, 27100 Pavia (IT)
(72) Inventor: Marini, Giuseppe, 40064 Ozzano dell'Emilia (IT); Muggiasca, Andrea, 27100 Pavia (IT)
(74) Representative: Ruzzu, Giammario
(86) International application number: PCT/IB2014/065581
(87) International publication number: WO 2015/059666

(56) References cited:
- CA-A1- 2 359 232
- JP-A- H03 202 070
- US-A1- 2004 062 694
- US-A1- 2008 183 181

## Description

### TECHNICAL FIELD

The present invention relates to the technical field concerning the production of pipes of plastic material, mainly HD polyethylene, but also PVC (polyvinyl chloride) or polypropylene, used in the hydro-thermo-sanitary plants.

### BACKGROUND ART

It is known that nowadays hydraulic plants intended to deliver drinking water for civil or industrial uses or heating/cooling plants, are made predominantly with pipes of plastic material, such as HD polyethylene, PVC or polypropylene. The pipes made of these materials offer considerable advantages with respect to metallic pipes (steel, copper or, historically, lead), which were used in previous time. Actually, the pipes of plastic material, available nowadays, offer excellent resistance to mechanical stress and chemical agents, last a long time without damaging and are simple and easy to install.

The most common material used for the above mentioned pipes is high density polyethylene (HDPE), which is unanimously considered the most suitable material for applications in which contact with water for human use is foreseen. The same material is used also in waterworks field, for the production of pipes, usually of large diameter, intended for use in drinking water distribution systems.

Moreover, the hydro-thermo-sanitary applications use advantageously multilayer composite pipes. In such pipes, two or more layers of polyethylene are alternated with layers of material adapted to constitute a barrier for oxygen, since the polyethylene is somewhat permeable to this element.

In order to improve their mechanical resistance (and in particular the time regression curve), flexibility and resistance to corrosion, the pipes made of HD polythene are preferably subjected to a cross-linking process, during which chemical bonds are created among the various polymeric chains or among various points of the same chain. The ways to obtain the chemical reaction that produces cross-linking, as well as the advantages deriving therefrom, have been well known for a long time to those skilled in the art.

A known technique of cross-linking of pipes of HD polythene used in a hydro-thermo-sanitary field includes the treatment of the pipes inside an autoclave facility with saturated steam, in controlled temperature and pressure conditions. In accordance with such technique, the pipes, roll- or bar-shaped, are introduced into an autoclave and kept there for a predetermined period of time in the above mentioned controlled temperature and pressure conditions.

The degree of cross-linking of HD polyethylene that can be obtained with the above mentioned treatment is a function of the thickness of the pipes walls, temperature and pressure inside the autoclave and the duration of the stay in such conditions. Typically, the temperature values can reach more than 130°C, the pressure values can reach 3 bar and the duration of the stay can be comprised between 20 and 120 minutes.

### TECHNICAL PROBLEM

As far as the hydraulic plants for the distribution of drinking water are concerned, as well as for the distribution of domestic hot water, supplying swimming pools, spa and other wellness centres, the problems related to obtaining and maintaining of suitable hygienic and sanitary conditions inside the water transport and distribution conduits have always been treated with great concern. Under this aspect, there are well known techniques, equipment and products for reaching and maintaining acceptable levels of microbial population circulating in the water flow and of the bacterial film, which inevitably forms in the plants, and especially in their parts subjected to water stagnation or to limited exchange.

The known sanitization techniques are mainly based on the use of chlorine and its compounds, especially hypochlorites or chloramines, which are introduced in circulation at very precise concentrations.

For some time, more and more care has been taken of the hygienic-sanitary situation of the components of the above mentioned plants, and in particular of the various pipes and fittings, during their installation. Actually, it is important to take into consideration that since their production until their installation, the pipes of HD polyethylene are kept in completely non-protected environments, such as storehouses of factories, wholesale dealers and retailers. Moreover, once bought, they can stay for days, weeks or months in the installation sites. Consequently, the pipes and other components are subjected to the action of air, dust and often to severe weather conditions for a long time. Moreover, their conformation makes the pipes prone to colonization by various kinds of insects and other animals, even reptiles and rodents, at least the pipes of bigger diameter. Such organisms can make nests, encrustations or in any case dirt of various kinds inside the pipes.

In any event, when they are installed, the pipes and other components of the hydro-thermo-sanitary plants completely lack the most fundamental hygiene requirements and the presence of insects and microorganisms thereinside is wholly out of control.

This situation can make the subsequent sterilization of the hydraulic plant more complicated and less efficacious and in any case, it is harmful for the overall hygiene of the same plant.

### OBJECTS OF THE INVENTION

The object of the present invention is to propose a method for improving the hygienic and sanitary characteristics of the hydraulic components destined to be used in the hydro-thermo-sanitary plants, and in particular of the pipes and fittings made of plastic materials, such as HD polyethylene, PVC or polypropylene.

Another object of the invention is to maintain the so obtained hygienic and sanitary characteristics for a long time, preferably until the hydraulic components are installed in a plant.

A further object of the invention is to allow the identification of the above mentioned hydraulic components, whose above mentioned improved hygienic and sanitary characteristics are no longer verified during their installation.

A yet further object of the invention is to propose a hydraulic component, which has the above mentioned hygienic and sanitary characteristics, when it leaves the production line and which is capable of maintaining them for a long time in the absence of manipulations.
make nests, encrustations or in any case dirt of various kinds inside the pipes.

In any event, when they are installed, the pipes and other components of the hydro-thermo-sanitary plants completely lack the most fundamental hygiene requirements and the presence of insects and microorganisms thereinside is wholly out of control.

This situation can make the subsequent sterilization of the hydraulic plant more complicated and less efficacious and in any case, it is harmful for the overall hygiene of the same plant.

Sterilization techniques for components are known in other fields, typically in the medical field. It is known, for example, that syringes and catheters can be sterilized by placing them in under the effect of a sterilizing gas within containers that are permeable to that gas. These containers are generally formed by limp bags, which are entirely or partially made from material permeable to the sterilizing gas and are big enough to hold the components thereinside.
in particular, the US 2008/0183181 (Kevin Treacy et al.) discloses a device for treatment of heart diseases including a catheter with a coupling element at an end. The catheter is enclosed within a container that is partially made from material permeable to the sterilizing gas. The container of the US 2008/0183181 features an elongated element with a wider end, or both ends, for receiving the coupling element.

Although the US 2008/0183181 addresses the problem of sterilizing an object, the technical field for which the technical problem is resolved is far distant from the technical field of the present invention, which is related to hydraulic components intended for hydro-thermo-sanitary plants. The inventors of such components and production techniques are therefore not expected to have knowledge of production techniques common in the medical device industry, especially in the case in which the only common feature between the latter and the components for hydro-thermo-sanitary use consists in the their elongated shape. For these reasons, the solutions adopted in the medical field may not tout-court become part of the state of the art for the technology of hydro-thermo-sanitary components.

### OBJECTS OF THE INVENTION

The object of the present invention is to propose a method for improving the hygienic and sanitary characteristics of the hydraulic components destined to be used in the hydro-thermo-sanitary plants, and in particular of the pipes and fittings made of plastic materials, such as HD polyethylene, PVC or polypropylene.

Another object of the invention is to maintain the so obtained hygienic and sanitary characteristics for a long time, preferably until the hydraulic components are installed in a plant.

A further object of the invention is to allow the identification of the above mentioned hydraulic components, whose above mentioned improved hygienic and sanitary characteristics are no longer verified during their installation.

A yet further object of the invention is to propose a hydraulic component, which has the above mentioned hygienic and sanitary characteristics, when it leaves the production line and which is capable of maintaining them for a long time in the absence of manipulations.

### SUMMARY OF THE INVENTION

The above mentioned objects are wholly obtained by a method for improving the hygienic and sanitary characteristics of a hydraulic component, such as a pipe or fitting of plastic material, made in accordance with the contents of the independent claim 1, and by a hydraulic component, such as a pipe or fitting of plastic material, made in accordance with the contents of the independent claim 4. The method of the invention is aimed at improving the hygienic and sanitary characteristics of hydraulic components intended to be installed in a hydro-thermo-sanitary plant or in waterworks. Each of the above mentioned hydraulic components comprises in particular a tubular body and at least two inlet ports at the opposite ends of the same.

The method includes the following operating steps: applying a removable protective cover to each of said inlet ports of the hydraulic component, for preventing entrance of impurities, organisms and biological agents into the tubular body; introducing the hydraulic component into an autoclave facility; carrying out a cycle of treatment of the hydraulic component in controlled temperature and pressure, inside the autoclave facility, for a predetermined period of time; taking the hydraulic component out from said autoclave facility.

The hydraulic component having improved hygienic and sanitary characteristics, proposed by the invention, such as a pipe destined for hydro-thermo-sanitary uses or in waterworks, is provided with a removable cover and subjected to the above described method.

### BRIEF DESCRIPTION OF THE DRAWINGS

The characteristics of the invention, as they will become evident from the claims, are pointed out in the following detailed description, with reference to the enclosed table of drawings, in which:
- Figure 1 is a schematic view of a component of a hydraulic plant and in particular of a piece of pipe in the form of roll, made according to the present invention;
- Figure 2 shows, in enlarged scale, the portion A of the piece of pipe of Figure 1, according to a first embodiment of the invention;
- Figure 3 is a schematic view of a removable cover made according to the embodiment of Figure 2, in an open configuration;
- Figure 4 is the same view as Figure 2, in another embodiment of the invention.

### DESCRIPTION OF PREFERRED EMBODIMENTS OF THE INVENTION

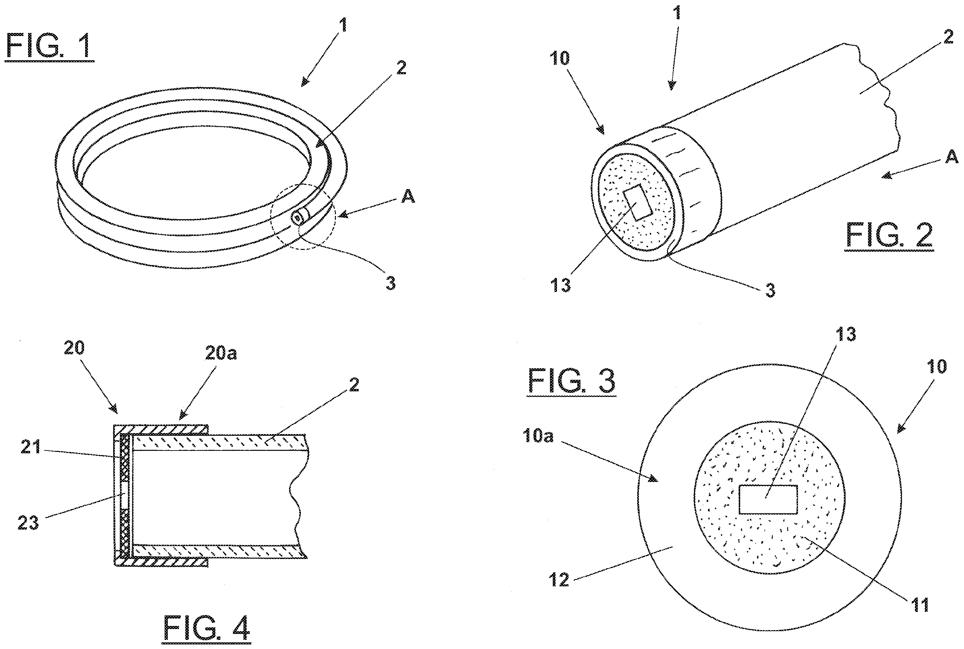

With reference to Figures 1 to 3 and a first, but not only embodiment of the invention, reference numeral 1 indicates a hydraulic component intended to be installed in a hydro-thermo-sanitary plant. The figure illustrates a piece of rolled pipe as an example. However, it is to be understood that what will be described later on, will refer also to other hydraulic components, for example two-, three-way fittings, sleeves, etc.

Furthermore, the pipes proposed by the invention can also be of the type supplied in bars of predetermined length, including those of larger diameter, intended to be used in waterworks, for the production of drinking water distribution system.

The piece of pipe 1 is made of a suitable plastic material and preferably of HD polyethylene for reasons which will become clear from the following description. In any case, with regard to the invention, the pipe 1 can be made also of different materials, such as PVC, polypropylene, etc.

For the same reasons, it is advantageous and suitable, for the purposes of carrying out of the invention, that the above mentioned piece of pipe 1 be subjected to cross-linking treatment in an autoclave, in controlled temperature and pressure and in the presence of saturated steam, aimed at improving its mechanical characteristics and resistance to corrosion, according to a conventional production process.

The piece of pipe 1 comprises a tubular body 2 and is provided, at its ends, with two inlet ports 3, only one of which is seen in the rolled form of the body in Figure 1.

Preferably, immediately after that the pipe leaves the production line and after that the piece 1 is cut, a removable protective cover 10 is applied to each of the above mentioned inlet ports 3. The removable protective cover 10 is permeable to steam and, at the same time, when the treatment proposed by the invention is completed, it provides a barrier for impurities and organisms and biological agents inside the tubular body 2. The term organisms means both insects, reptiles or other macroorganisms, and bacteria or other microorganisms.

In particular, according to the first embodiment of the invention, the above mentioned protective cover 10 is constituted by a shaped sheet (in this case substantially circular) 10a, made of a suitable yielding material.

Figure 3 illustrates the protective cover 10 in its open configuration, before it is mounted on the relative terminal portion of the pipe 1.

A central portion 11 of the shaped sheet 10a, intended to be positioned in correspondence to the inlet port 3, is made of a material permeable to a sterilizing agent, which can be constituted by water steam or by ethylene oxide. Such materials are well known and commercially available, since they are commonly used to produce bags, or parts thereof, for containing things to be sterilized, for example, in medical or pharmaceutical field, such as syringes, catheters, etc. In particular, the shaped sheet 10a can be made of the *"medical grade"* paper material, of the product commercially known as *"tyvek"* or of materials having equivalent properties.

The outermost ring 12 of the shaped sheet 10a, which surrounds the central portion 11, is made auto adhesive on its surface by means of a suitable surface treatment, using wholly known techniques, in such a way as to allow the cover 10 to be fixed to the tubular body 2 by adhesion to the outer wall thereof.

In the central portion 11 there is also a control label 13, aimed at modifying its colour due to its contact with the above mentioned sterilizing agent.

A second embodiment of the invention, shown in Figure 4, provides a removable protective cover 20 for the pipe 1, constituted by a cap 20a, whose dimensions are suitable to allow its coupling with one end of the same pipe 1. The cap 20a in turn, comprises a ring 22, made of semi rigid or flexible plastic material and destined to house the end of the pipe 1 and to stabilize the interference or gluing coupling.

The central part 22 of the cap 20a, aimed at covering an inlet port 3 of the pipe 1, is made of the same material, permeable to a sterilizing agent, already described in relation to the first embodiment of the invention. In the aforesaid central portion 22 there is introduced also a control label 23, which modifies its colour due to its contact with the above mentioned sterilizing agent.

According to the above described method for improving and maintaining the hygienic and sanitary characteristics of the hydraulic component 1, for example of the piece of pipe, protective covers 10, 20, obtained in accordance with one of the above described embodiments, are applied to the inlet ports 3 of the hydraulic component. It is preferred, although not necessary, that such protective covers 10, 20 be applied directly after the piece of pipe 1 has been obtained in the production line.

As it has already been anticipated before, in the normal production process, it is preferred that the piece of pipe 1 be subjected to the cross-linking treatment in an autoclave in controlled temperature and pressure, in the presence of saturated steam. The parameters for carrying out such treatment are already known, in terms of the trend of values of temperature and pressure in the autoclave, as well as the duration of the stay thereinside.

In this case, the carrying out of the method of the invention partially overlaps such treatment. Actually, the method includes the introduction of the piece of pipe protected by the protective covers 10 into the autoclave and carrying out a treatment in controlled temperature and pressure, in the presence of a sterilizing agent, which can be validly constituted by saturated steam used to obtain the cross-linking of the piece of pipe 1.

For the purposes of the cross-linking treatment, the water steam can involve both the outer wall of the piece of pipe 1 and the inner one, by virtue of the permeability of the protective covers 10 to the steam. Therefore, the success of the cross-linking treatment is not invalidated by the presence of the protective covers 10.

For the purposes of the invention, the carrying out of the above described treatment is necessary for improving the hygienic and sanitary characteristics inside the pipe 1. Essentially, due to such treatment it is possible to obtain a high sterilization degree inside the pipe 1. In fact, the temperatures that are normally reached in the autoclave (generally, higher than 120°C), together with the duration of the stay inside it (normally longer than 30 minutes) are sufficient to destroy almost all the microorganisms present thereinside.

However, for the purposes of the invention, it is not necessary to specify in what extent the microbial population must be killed. In fact, taking into consideration the subsequent use of the piece of pipe 1, for which a minimum certified sterility degree is certainly not required, as it happens for most critical products, such as medical ones, the required object is to ensure an improvement of the hygienic and sanitary characteristics inside the piece of the same pipe 1 and especially, their maintenance for a long period of time, after they have been removed from the autoclave.

According to the invention, a considerable improvement of the hygienic and sanitary characteristics inside the pipe 1 is ensured by the heat treatment and by the presence of the sterilizing agent inside the tubular body 2. Carrying out of the treatment can be confirmed by the change of colour of the control label 13.

The maintenance of such characteristics over time is also advantageously guaranteed, even during the installation of the piece of pipe in a hydro-thermo-sanitary hydraulic plant, by the integrity of the above mentioned protective covers.

If the use of saturated steam is not necessary in the cross-linking treatment, or if the method according to the invention is applied in the absence of such treatment, the sterilizing agent can be constituted also by ethylene oxide, which is commonly used in the medical-pharmaceutical field.

It is understood that what above has been described as a pure not limiting example. Therefore, possible changes and variants of the invention are considered to be within the protective scope granted to the present technical solution, as described above and claimed below.

## Claims

1. A method for the improvement of the hygienic and sanitary characteristics of a hydraulic component intended to be installed in a hydro-thermo-sanitary plant or in waterworks, said hydraulic component (1) comprising a piece of pipe of plastic material or a fitting of the same material, said piece of pipe or fitting (1) including a tubular body and at least two inlet ports (3) at opposite ends of said tubular body, said method being **characterized by** including the following operating steps: applying a removable protective cover (10) to each of said inlet ports (3) of said hydraulic component, for preventing entrance of impurities, organisms and biological agents into said tubular body (2) of the same; introducing said hydraulic component (1) into an autoclave facility; carrying out a cycle of treatment of said hydraulic component (1) at controlled temperature and pressure, inside said autoclave facility, for a predetermined period of time so as to improve the hygienic and sanitary characteristics of the inner parts of the component (1) by virtue of the permeability of the removable protective covers (10) while subjecting said hydraulic component (1) to the action of a sterilizing agent during said cycle of treatment at controlled temperature and pressure, at least part of said protective cover (10) applied to the inlet ports (3) of the same being permeable to said sterilization agent; taking said hydraulic component (1) out from said autoclave facility.

2. A method as claimed in claim 1, **characterized in that** said sterilizing agent is formed by saturated steam.

3. A method as claimed in claim 1, **characterized in that** said sterilizing agent is formed by ethylene oxide.

4. A hydraulic component intended to be installed in a hydro-thermo-sanitary plant or in waterworks, said hydraulic component (1) including a piece of pipe of plastic material or a fitting of the same material, said piece of pipe or fitting (1) including a tubular body (2) and at least two inlet ports (3) at the opposite ends of said tubular body (2), including, a barrier for preventing entrance of air, impurities and organisms into said tubular body (2) and for ensuring that carrying out a cross-linking treatment cycle for said hydraulic component (1) in controlled temperature and pressure, inside an autoclave facility, improves the hygienic and sanitary characteristics present inside said tubular body (2); **characterized in that** said barrier consists of a removable protective cover (10,20) for each of said inlet ports (3), said protective cover in turn including at least one central portion (11,21) permeable to a sterilizing agent, positioned in correspondence to the inlet port (3).

5. A hydraulic component as claimed in claim 4, **characterized in that** each of said removable protective covers (10) includes a shaped sheet (10a) of plastic material, at least one outer circle crown (12) of the same being adhesive, to allow attachment thereof to the terminal portion of the cited hydraulic component (1), said portion (11), permeable to a sterilizing agent, being situated in correspondence to the relative above mentioned inlet port (3).

6. A hydraulic component as claimed in claim 4, **characterized in that** each of said removable protective covers (20) includes a cap of plastic material (20a), adapted to be fastened to the end portion of said hydraulic component (1) by interference or by gluing, said portion (21), permeable to a sterilizing agent, being situated in correspondence to the relative above mentioned inlet port (3).

7. A hydraulic component as claimed in any of claims 4 to 6, **characterized in that** at least one of the removable protective covers (10, 20) applied to each said hydraulic component (1) includes a central control label (13, 23), adapted to undergo a colour change when a treatment default temperature is reached or due to the contact with the said sterilization agent, for providing an indication of the completion of the treatment of the relative hydraulic component (1).

8. A hydraulic component as claimed in any of claims 4 to 6, **characterized in that** said central portion (11,21) is permeable to water steam, said sterilizing agent is formed by saturated steam.

9. A hydraulic component as claimed in any of claims 4 to 6, **characterized in that** said central portion (11,21) is permeable to ethylene oxide, said sterilizing agent being formed by ethylene oxide.

## Patentansprüche

1. Verfahren zur Verbesserung der hygienischen und sanitären Eigenschaften einer Hydraulikkomponente, die für den Einbau in eine Wasser-, Heizungs- und Sanitäranlage oder in Wasserwerke vorgesehen ist, wobei die Hydraulikkomponente (1) ein Rohrstück aus Kunststoff oder einen Einsatz aus dem gleichen Material umfasst, und wobei das Stück Rohr oder Einsatz (1) einen Rohrkörper und mindestens zwei Einlassöffnungen (3) an gegenüberliegenden Enden des Rohrkörpers beinhaltet, das Verfahren **dadurch gekennzeichnet ist, dass** es die folgenden Betriebsschritte beinhaltet: eine abnehmbare Schutzabdeckung (10) an jeder der Einlassöffnungen (3) der Hydraulikkomponente angebracht ist, um das Eindringen von Verunreinigungen, Organismen und biologischen Mitteln in den Rohrkörper (2) derselben zu verhindern; die Hydraulikkomponente (1) in eine Autoklavenanlage eingebracht wird; ein Behandlungszyklus der Hydraulikkomponente (1) bei kontrollierter Temperatur und kontrolliertem Druck innerhalb der Autoklavenanlage für einen vorbestimmten Zeitraum durchgeführt wird, um die hygienischen und hygienischen Eigenschaften der inneren Teile der Komponente (1) aufgrund der Durchlässigkeit der abnehmbaren Schutzabdeckungen (10), während die Hydraulikkomponente (1) während des Behandlungszyklus bei kontrollierter Temperatur und kontrolliertem Druck der Wirkung eines Sterilisationsmittels ausgesetzt wird, zu verbessern, wobei mindestens ein Teil der auf die Einlassöffnungen (3) derselben aufgebrachten Schutzabdeckung (10) für das Sterilisationsmittel durchlässig ist; die Hydraulikkomponente (1) aus der Autoklavenanlage entnommen wird.

2. Verfahren nach Anspruch 1, **dadurch gekennzeichnet, dass** es sich bei dem Sterilisiermittel um Sattdampf handelt.

3. Verfahren nach Anspruch 1, **dadurch gekennzeichnet, dass** es sich bei dem Sterilisiermittel um Ethylenoxid handelt.

4. Hydraulikkomponente, die für den Einbau in eine Wasser-, Heizungs- und Sanitäranlage oder in Wasserwerke vorgesehen ist, wobei die Hydraulikkomponente (1) ein Rohrstück aus Kunststoff oder einen Einsatz aus dem gleichen Material umfasst, und wobei das Stück Rohr oder Einsatz (1) einen Rohrkörper (2) und mindestens zwei Einlassöffnungen (3) an gegenüberliegenden Enden des Rohrkörpers (2) beinhaltet, die eine Barriere enthalten, um das Eindringen von Luft, Verunreinigungen und Organismen in den Rohrkörper (2) zu verhindern, und die Durchführung eines Vernetzungsbehandlungszyklus für die Hydraulikkomponente (1) bei kontrollierter Temperatur und Druck innerhalb einer Autoklavenanlage die im Inneren des Rohrkörpers (2) vorhandenen hygienischen und hygienischen Eigenschaften verbessern;
**dadurch gekennzeichnet, dass** die Barriere aus einer abnehmbaren Schutzabdeckung (10, 20) für jede der Einlassöffnungen (3) besteht, wobei die Schutzabdeckung wiederum mindestens einen zentralen Abschnitt (11, 21) beinhaltet, der für ein Sterilisiermittel durchlässig ist und entsprechend der Einlassöffnung (3) angeordnet ist.

5. Hydraulikkomponente nach Anspruch 4, **dadurch gekennzeichnet, dass** jede der abnehmbaren Schutzabdeckungen (10) eine geformte Folie (10a) aus Kunststoff beinhaltet, wobei mindestens eine äußere Kreiskrone (12) derselben klebend ist, um deren Befestigung an dem Endabschnitt der genannten Hydraulikkomponente (1) zu ermöglichen, und der Abschnitt (11), der für ein Sterilisiermittel durchlässig ist, in Übereinstimmung mit der relativen oben genannten Einlassöffnung (3) angeordnet ist.

6. Hydraulikkomponente nach Anspruch 4, **dadurch gekennzeichnet, dass** jede der abnehmbaren Schutzabdeckungen (20) eine Kappe aus Kunststoff (20a) beinhaltet, die um am Endabschnitt der Hydraulikkomponente (1) durch Einklemmen oder Kleben befestigt zu werden geeignet ist, und der Abschnitt (21), der für ein Sterilisiermittel durchlässig ist, in Übereinstimmung mit der relativen oben genannten Einlassöffnung (3) angeordnet ist.

7. Hydraulikkomponente nach einem der Ansprüche 4 bis 6, **dadurch gekennzeichnet, dass** mindestens eine der abnehmbaren Schutzabdeckungen (10, 20), die auf jede der Hydraulikkomponenten (1) aufgebracht sind, ein zentrales Steuerungsschild (13, 23) aufweist, das so angepasst ist, dass es bei Erreichen einer Behandlungsstandardtemperatur oder aufgrund des Kontakts mit dem Sterilisationsmittel eine Farbänderung erfährt, um einen Hinweis auf den Abschluss der Behandlung der jeweiligen Hydraulikkomponente (1) zu geben.

8. Hydraulikkomponente nach einem der Ansprüche 4 bis 6, **dadurch gekennzeichnet, dass** der zentrale Abschnitt (11, 21) für Wasserdampf durchlässig ist, das Sterilisiermittel durch Sattdampf gebildet wird.

9. Hydraulikkomponente nach einem der Ansprüche 4 bis 6, **dadurch gekennzeichnet, dass** der zentrale Abschnitt (11, 21) für Ethylenoxid durchlässig ist, wobei es sich bei dem Sterilisiermittel um Ethylenoxid handelt.

## Revendications

1. Procédé pour l'amélioration des caractéristiques hygiéniques et sanitaires d'un composant hydraulique qui est destiné à être installé dans une installation hydro-thermo-sanitaire ou dans une usine hydraulique, ledit composant hydraulique (1) comprenant un morceau de tuyau en matière plastique ou un raccord du même matériau, ledit morceau de tuyau ou raccord (1) comprenant un corps tubulaire et au moins deux orifices d'entrée (3) aux extrémités opposées dudit corps tubulaire, ledit procédé étant caractérisé en ce qu'il comprend les étapes de fonctionnement suivantes: un couvercle protecteur amovible (10) est appliqué à chacun desdits orifices d'entrée (3) dudit composant hydraulique, pour empêcher l'entrée d'impuretés, d'organismes et d'agents biologiques dans ledit corps tubulaire (2) de celui-ci; ledit composant hydraulique (1) est introduit dans une installation d'autoclave; un cycle de traitement dudit composant hydraulique (1) est effectué à température et pression contrôlées, à l'intérieur de ladite installation d'autoclave, pendant une durée prédéterminée afin d'améliorer les caractéristiques hygiéniques et sanitaires des parties internes du composant (1) grâce à la perméabilité des capots de protection amovibles (10) en soumettant ledit composant hydraulique (1) à l'action d'un agent stérilisant pendant ledit cycle de traitement à température et pression contrôlées, au moins une partie dudit couvercle protecteur (10) appliqué aux orifices d'entrée (3) de celui-ci étant perméable audit agent de stérilisation;
ledit composant hydraulique (1) est retiré de ladite installation d'autoclave.

2. Procédé selon la revendication 1, **caractérisé en ce que** ledit agent stérilisant est formé par de la vapeur saturée.

3. Procédé selon la revendication 1, **caractérisé en ce que** ledit agent stérilisant se compose d'oxyde d'éthylène.

4. Composant hydraulique destiné à être installé dans une installation hydro-thermo-sanitaire ou dans une usine hydraulique, ledit composant hydraulique (1) comprenant un morceau de tuyau en matière plastique ou un raccord du même matériau, ledit morceau de tuyau ou raccord (1) comprenant un corps tubulaire (2) et au moins deux orifices d'entrée (3) aux extrémités opposées dudit corps tubulaire (2), comprenant une barrière pour empêcher l'entrée d'air, d'impuretés et d'organismes dans ledit corps tubulaire (2) et pour s'assurer que la réalisation d'un cycle de traitement de réticulation dudit composant hydraulique (1) à température et pression contrôlées, à l'intérieur d'un installation d'autoclave, améliore les caractéristiques hygiéniques et sanitaires présentes à l'intérieur dudit corps tubulaire (2);
**caractérisé en ce que** ladite barrière consiste en un couvercle protecteur amovible (10, 20) pour chacun desdits orifices d'entrée (3), ledit couvercle protecteur comprenant à son tour au moins une partie centrale (11, 21) perméable à un agent stérilisant, qui est positionnée en correspondance avec l'orifice d'entrée (3).

5. Composant hydraulique selon la revendication 4, **caractérisé en ce que** chacun desdits couvercles de protection amovibles (10) comprend une feuille profilée (10a) en matière plastique, au moins une couronne de cercle extérieur (12) de celle-ci étant adhésive, pour permettre sa fixation sur la partie terminale du composant hydraulique cité (1), ladite partie (11), qui est perméable à un agent stérilisant, étant située en correspondance avec l'orifice d'entrée relatif mentionné ci-dessus (3).

6. Composant hydraulique selon la revendication 4, **caractérisé en ce que** chacun desdits couvercles de protection amovibles (20) comprend un capuchon en matière plastique (20a), adapté pour être fixé à la partie d'extrémité dudit composant hydraulique (1) par interférence ou par collage, ladite partie (21), qui est perméable à un agent stérilisant, étant située en correspondance avec l'orifice d'entrée relatif mentionné ci-dessus (3).

7. Composant hydraulique tel que revendiqué dans l'une quelconque des revendications 4 à 6, **caractérisé en ce que** au moins un des couvercles de protection amovibles (10, 20) appliqués sur chacun desdits composants hydrauliques (1) comporte une étiquette de commande centrale (13, 23), qui est adaptée pour subir un changement de couleur lorsqu'une température de défaut de traitement est atteinte ou en raison du contact avec ledit agent de stérilisation, pour fournir une indication de l'achèvement du traitement du composant hydraulique relatif (1).

8. Composant hydraulique tel que revendiqué dans l'une quelconque des revendications 4 à 6, **caractérisé en ce que** ladite partie centrale (11, 21) est perméable à la vapeur d'eau, ledit agent stérilisant se compose de vapeur saturée.

9. Composant hydraulique tel que revendiqué dans l'une quelconque des revendications 4 à 6, **caractérisé en ce que** ladite partie centrale (11, 21) est perméable à l'oxyde d'éthylène, ledit agent stérilisant étant formé par de l'oxyde d'éthylène.
